Europäisches Patentamt

**European Patent Office**

**Office européen des brevets**

(11) Numéro de publication: **0 035 086**
**A1**

## DEMANDE DE BREVET EUROPEEN

(21) Numéro de dépôt: **80810352.7**

(22) Date de dépôt: **14.11.80**

(51) Int. Cl.³: **G 09 B 19/16, A 61 B 5/18**

(30) Priorité: **22.02.80 CH 1429/80**

(43) Date de publication de la demande: **09.09.81**
**Bulletin 81/36**

(84) Etats contractants désignés: **AT BE CH DE FR GB IT LI LU NL SE**

(71) Demandeur: **Gurtner, Michel, 18B, chemin François Chavaz, CH-1213 Onex (CH)**

(72) Inventeur: **Gurtner, Michel, 18B, chemin François Chavaz, CH-1213 Onex (CH)**

(74) Mandataire: **Moinas, Michel, c/o MOINAS & CIE Case Postale 88, CH-1211 Genève 4 (CH)**

(54) **Installation pour apprentissage et examens de conduite automobile.**

(57) Cette installation comprend un espace disponible (H) et une largeur de parcours (G) réglées en fonction du véhicule à manœuvrer à l'aide des éléments mobiles (C, D, E et F), les éléments (A, A1, B et B1) étant fixes. Des moteurs électriques (1) fixés à des murs (2) actionnent des axes (3) chargés de déplacer les éléments mobiles; ces derniers comprennent des glissières de sécurité (4, 5a et 5b). Des lignes continues (6, 6a et 6b) sont marquées sur le sol et indiquent la position optimale du véhicule en fin de manœuvre. Des capteurs (8 et 9) sont matérialisés au sol par des lignes discontinues. Un capteur de position (11) signale à quelle distance se trouve le véhicule.

Application à l'apprentissage et aux examens de conduite, pour parcages perpendiculaires et latéraux, tourner sur route et marche arrière, en particulier en virage.

# INSTALLATION POUR APPRENTISSAGE ET
## EXAMENS DE CONDUITE AUTOMOBILE

La présente invention a pour objet une installation de type électro-mécanique destinée à l'apprentissage et aux examens de conduite automobile, en ce qui concerne notamment les parcages perpendiculaires, les parcages latéraux, le tourner sur route et la marche arrière.

On connait les désavantages des méthodes actuelles qui se résument en trois points: premièrement, les dimensions des cases réservées aux parcages et la largeur des passages où circulent les véhicules ne correspondent pas exactement aux dimensions et caractéristiques de chaque véhicule. Il arrive ainsi que l'examinateur demande au candidat manoeuvrant une grande voiture d'effectuer un parcage dans une petite case ou vice versa. Deuxièmement, la tâche de l'examinateur de conduite est de vérifier les capacités du candidat à l'examen; trop souvent son appréciation est partiale, variable, imprécise, subjective et surtout approximative. Troisièmement, le candidat qui a peur, qui a le trac ou trouve l'examinateur antipathique perd ses moyens et échoue alors qu'il aurait les capacités de réussir.

Pour atténuer ou éliminer ces facteurs subjectifs, l'invention propose une installation de type électro-mécanique qui donne un compte-rendu exact et non-subjectif des manoeuvres effectuées. Cette installation pour apprentissage et examens de conduite automobile est caractérisée en ce qu'elle comprend un espace de dimensions réglables, adaptable au véhicule devant l'emprunter, des éléments, délimitant cet espace, munis de détecteurs de présence, des capteurs de position, des récepteurs de signaux activant ou désactivant les détecteurs et les capteurs, et enfin des moyens d'enregistrement des données fournies par les détecteurs, capteurs et récepteurs.

De préférence, les éléments délimitant l'espace

0035086

sont d'abord des glissières de sécurité; certaines sont fixes, d'autres mobiles, portées par des axes mus, par exemple par des moteurs électriques de façon à permettre le réglage des dimensions de l'espace à emprunter par le véhicule en manoeuvre. Les détecteurs de présence peuvent être des pare-chocs montés sur dynamomètres; les capteurs de position et les récepteurs de signaux sont avantageusement solidaires eux-aussi sur les glissières de sécurité. Les récepteurs de signaux correspondant par exemple à des émetteurs du genre télécommande (sonique, ultrasonique ou infra-rouge) permettent au candidat d'activer l'installation lorsque la manoeuvre va débuter, et cela sans descendre du véhicule.

Les éléments délimitant l'espace peuvent être ensuite, pour certains, des éléments immatériels, tel qu'un faisceau optique du type oeil électronique par exemple, qui, de préférence, permettent la mise en marche d'un chronomètre de façon à mesurer le temps mis pour effectuer la manoeuvre. Ce chronomètre est arrêté, par exemple par télécommande, depuis le véhicule, lorsque le candidat estime que sa manoeuvre est terminée. Le chronomètre peut être aussi automatiquement arrêté en cas de fausse manoeuvre ou d'accrochage.

De préférence, on enregistrera un maximum de paramètres, tels que le début et la fin de chaque manoeuvre, ainsi que le temps mis pour le réaliser, les phases intermédiaires, la position du véhicule, les chocs et les accrochages, et également les dimensions de l'espace alloué au véhicule pour sa manoeuvre et la taille du véhicule. Ces paramètres seront enregistrés électroniquement, puis présentés de façon numérique ou graphique par exemple sur une fiche ou une carte.

Les avantages de l'installation sont multiples: le candidat est libre de commencer sa manoeuvre quand il le désire, en utilisant la télécommande; il est seul, donc calme, toujours lucide et confiant parce qu'il s'attend à recevoir une appréciation objective, impartiale, logique et exacte. Cette installation autorise l'appréciation des parcages perpendiculaires ou en épi, des parcages latéraux ou en créneau, du tourner sur route ou demi-tour et de la marche arrière, en particulier en virage.

L'invention sera mieux comprise en référence au dessin annexé donné à titre d'exemple.

La figure 1 est une vue en perspective d'une télécommande.

La figure 2 est une configuration de l'installation pour les parcages perpendiculaires.

La figure 3 est une configuration de l'installation pour les parcages latéraux.

La figure 4 est une configuration de l'installation pour le tourner sur route.

La figure 5 est une configuration de l'installation pour la marche arrière en virage.

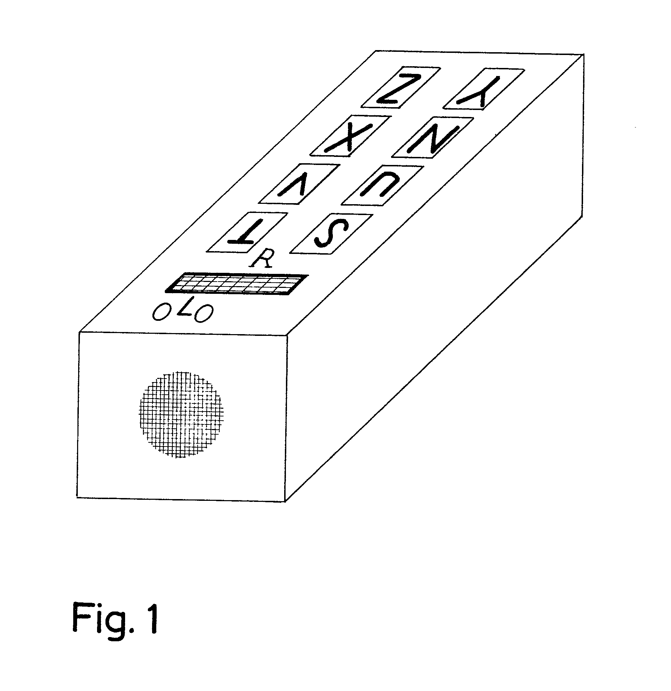

Dans la figure 1, on a représenté une télécommande à huit touches, donc huit fonctions, portant les références S à Z, servant, par l'intermédiaire des récepteurs de signaux, à mettre en marche et ou à arrêter l'enregistrement électronique des manoeuvres; cette télécommande comprend un appareil radio R permettant de communiquer à distance avec le local d'enregistrement. Deux lampes L témoins rouge et verte renseigne le candidat sur le fonctionnement de l'enregistrement.

En complément, non réprésenté spécifiquement sur la figure 1, la télécommande comprend aussi un chronographe mesurant le temps de manoeuvre TM et le temps total TT. Ce temps total TT réunit les temps TM de chaque manoeuvre, notamment les manoeuvres décrites ci-après et par exemple un démarrage en côte, ainsi que les temps de déplacement d'une aire de manoeuvre à l'autre. Ce temps total TT a un intérêt, car, en règle générale, un temps maximum est alloué pour l' examen. Le temps de manoeuvre TM s'arrête lorsque la manoeuvre est finie, l'élève ou le candidat l'indiquant en actionnant la touche correspondante S à Z. Il se remet automatiquement à zéro quand débute une autre manoeuvre, c'est-à-dire quand par exemple une des touches S à Z est actionnée à nouveau. Le temps total TT est remis à zéro entre chaque élève ou candidat.

Celui-ci est renseigné par le chronographe sur les temps TM et TT sans qu'il puisse en modifier les indications, indications qui sont enregistrées et présentées sur fiche ou carte.

En variante, le télécommande comprend un chronographe mesurant le temps de manoeuvre TM, le temps total TT et le temps de correction TC. Ce temps est important, surtout au stade de la formation, quand un premier essai de manoeuvre échoue et que l'élève doit la recommencer. Il peut ainsi se faire une idée de sa progression.

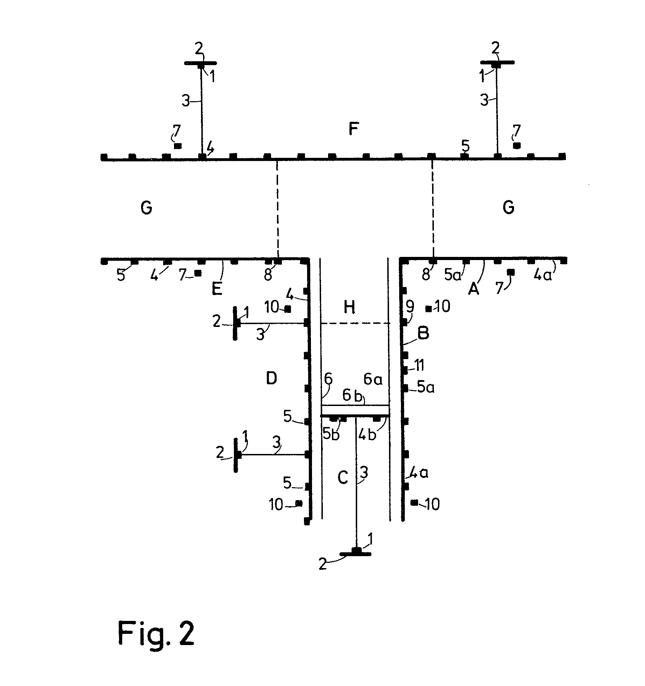

Dans la figure 2 pour parcages perpendiculaires, les dimensions de l'espace disponible et la largeur du parcage sont représentées par H et G respectivement. Elles sont réglées en fonction du véhicule à manoeuvrer à l'aide des éléments mobiles C, D, E et F, les éléments A, A1, B et B1 étant fixes. Des moteurs électriques 1 fixés à des murs 2 actionnent des axes 3 chargés de déplacer les éléments; ces derniers comprennent des glissières de sécurité 4, 4a et de pare-chocs 4b fixés à des supports 5, 5a et 5b. Des lignes

- 5 -    0035086

continues 6, 6a et 6b sont marquées sur le sol et indiquent la position optimale du véhicule en fin de manoeuvre. Des capteurs sont représentés en 8 et 9 et les lignes discontinues matérialisent ces capteurs au sol. Un capteur de position 11 signalera à quelle distance de la bordure ou d'une ligne continue se trouve le véhicule.

Avant d'effectuer le parcage, le candidat appuie sur la touche de la télécommande correspondant au parcage choisi en l'orientant contre un microphone 7 placé sur le bord du passage G; cette action provoque la mise en marche de l'enregistrement électronique qui passe de l'état d'attente à l'état réceptif. Dès lors, le candidat peut commencer le parcage, lorsque le véhicule parvient en 8 un capteur de présence met en marche un chronographe situé dans le local d'enregistrement, le début du parcage est enregistré et inscrit sur une carte; quand la moitié du véhicule est engagée dans l'espace, un capteur de présence 9 transmet l'information au chronographe, cette phase intermédiaire est inscrite sur la carte. Lorsque le candidat considère le parcage terminé, il appuie sur la touche de la télécommande correspondant au parcage en l'orientant contre un microphone 10 situé sur le côté de la case H, simultanément le chronographe sera arrêté et la position du véhicule est enregistrée par un capteur de position 11, la fin du parcage et la position du véhicule étant inscrites sur la carte.

Quatre parcages différents peuvent avoir lieu.

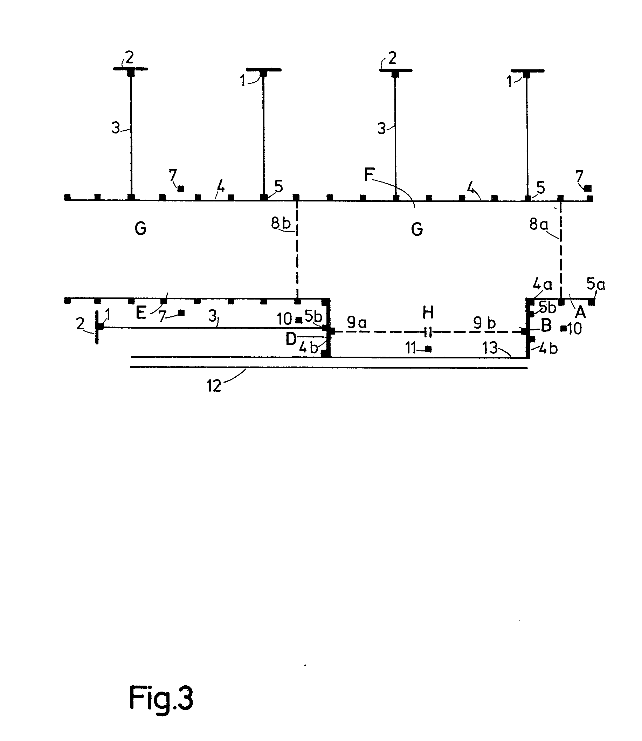

Dans la figure 3 pour parcages latéraux où les références ont la même signification que dans la figure 2, on a représenté en plus une bordure 13 et un détecteur par contact 12 qui indique si le véhicule franchit la bordure 13, cette information étant alors enregistrée. Le capteur 9a, comme le capteur 9b, détecte l'arrivée du véhicule dans la moitié arrière de l'espace de parcage.

Le candidat opère comme décrit plus haut.

- 6 -

0035086

L'enregistrement du parcage latéral en arrière à droite se fait par les capteurs 7, 8a, 9a, 10 et 11, l'enregistrement du parcage latéral en arrière à gauche par les capteurs 7, 8b, 9b, 10 et 11. A la fin du parcage lorsque le candidat appuie sur la touche de la télécommande en l'orientant contre le microphone 10, simultanément le chronographe est arrêté et la position du véhicule est enregistrée par le capteur de position 11; si le véhicule passe par dessus la bordure 13 et roule au-delà, un capteur de contact pur 12 le détecte et transmettra l'information à la table d'enregistrement.

Deux parcages différents (gauche ou droite) peuvent avoir lieu.

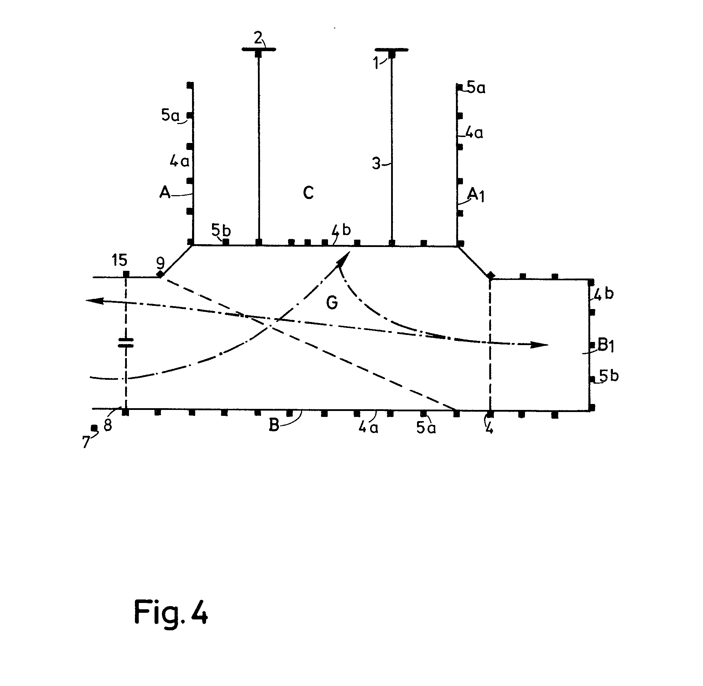

Dans la figure 4 pour le tourner sur route, les références ont la même signification que dans les figures précédentes.En trait mixte points-traits, on a de plus symbolisé la trajectoire du véhicule. Le début du tourner sur route est enregistré par un capteur de présence 8 qui met en marche le chronographe, puis d'autres enregistrements se succèdent par l'intermédiaire des capteurs de présence 9, 14 et 15, ce dernier capteur 15 enregistrant la fin du tourner sur route et arrêtant le chronographe.

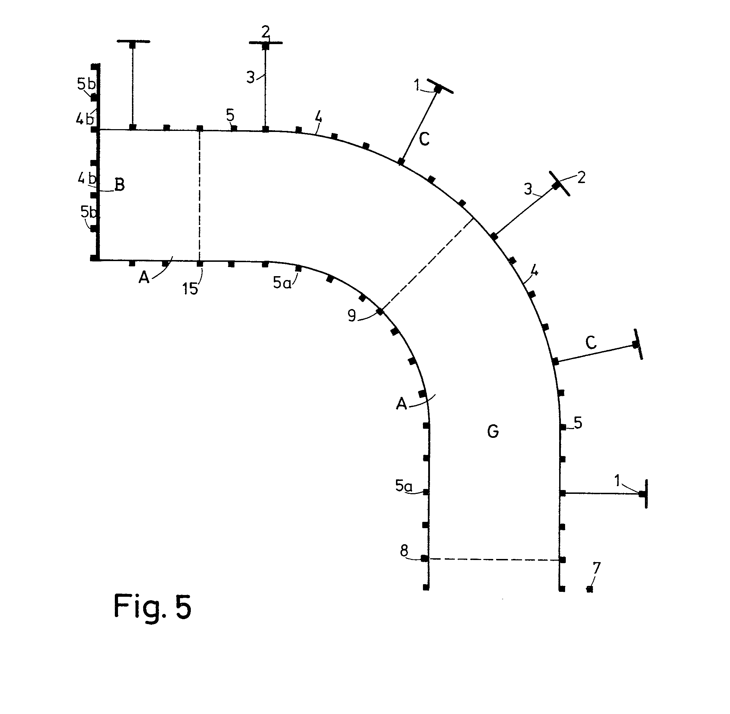

Dans la figure 5 pour la marche arrière en virage, avec les mêmes références que dans les figures précédentes, le candidat appuie sur la touche de la télécommande correspondant à la marche arrière en l'orientant contre le microphone 7. Dès que le véhicule recule, la manoeuvre est enregistrée par le capteur de présence 8 qui met automatiquement en marche le chronographe; vers le milieu du virage, un capteur de présence 9 enregistre le déplacement du véhicule; la fin de la marche arrière est enregistrée par un capteur de présence 15 qui arrête le chronographe. On peut remarquer que, pour la marche arrière en virage comme pour le tourner sur route, la télécommande est utilisée uniquement avant l'enregistrement effectif de chaque manoeuvre. Si un choc ou un accrochage intervient sur la place d'exercices durant la manoeuvre ou avant celle-ci contre une glissière ou un pare-chocs, il est détecté par des capteurs de contact pur ou des dynamomètres incorporés aux supports 5, 5a et 5b, puis transmis et inscrit sur la carte. Le candidat est informé par des lampes témoins ou autres signaux quand débute et prend fin la manoeuvre et si éventuellement des chocs et accrochages ont eu lieu.

L'invention se rapporte également à une installation-maquette dans laquelle les manoeuvres sont exécutées par l'intermédiaire d'une télécommande. Le conducteur se trouve alors dans ou devant un poste de télécommande qui actionne un véhicule modèle réduit, lequel se déplace sur l'installation-maquette.

Il peut alors s'agir d'un matériel éducatif pour adultes ou enfants, destiné par exemple à familiariser ces derniers avec l'exécution des manoeuvres. Il peut s'agir aussi d'un jeu ou d'un jouet dans lequel deux ou plusieurs personnes rivalisent et testent leur habileté, le vainqueur étant celui qui a correctement réalisé toutes les manoeuvres requises dans un minimum de temps.

0035086

REVENDICATIONS

1. Installation pour apprentissage et examens de conduite, caractérisée en ce qu'elle comprend un espace de dimensions réglables, adaptable au véhicule devant l'emprunter, des éléments, délimitant cet espace, munis de détecteurs de présence, des capteurs de position, des récepteurs de signaux activant ou désactivant les détecteurs et les capteurs, et, enfin des moyens d'enregistrement des données fournies par les détecteurs, capteurs et récepteurs.

2. Installation selon la revendication 1, caractérisée en ce que les éléments sont pour certains fixes et pour d'autres mobiles.

3. Installation selon la revendication 2, caractérisée en ce que les éléments mobiles sont portés par des axes mus par des moteurs.

4. Installation selon la revendication 1, caractérisée en ce que les éléments sont pour certains des glissières de sécurité.

5. Installation selon la revendication 1, caractérisée en ce que les éléments sont pour certains des éléments immatériels, notamment des faisceaux optiques ou électriques.

6. Installation selon la revendication 1, caractérisée en ce que les capteurs et les récepteurs sont solidaires des éléments délimitant l'espace.

7. Installation selon la revendication 1, caractérisée en ce qu'aux récepteurs correspond une télécommande qui peut agir depuis le véhicule.

8. Installation selon la revendication 1 ou 5, caractérisée en ce qu'automatiquement un chronomètre est mis en marche pour mesurer le temps de manoeuvre.

9. Installation selon la revendication 1 ou 8, caractérisée en

- 2 -

0035086

ce qu'on enregistre le début et la fin de la manoeuvre et le temps mis pour la réaliser, les phases intermédiaires, la position du véhicule, les chocs et les accrochages, les dimensions de l'espace alloué au véhicule pour sa manoeuvre et la taille du véhicule.

10. Installation selon la revendication 1, caractérisée en ce que l'enregistrement est électrique et est présenté numériquement ou graphiquement sur une fiche ou une carte.

11. Installation selon la revendication 1 à titre de jeu, jouet ou matériel éducatif.

Fig. 1

Fig. 2

Fig.3

Fig. 4

Fig. 5

## DOCUMENTS CONSIDERES COMME PERTINENTS

| Catégorie | Citation du document avec indication, en cas de besoin, des parties pertinentes | Revendication concernée |
|---|---|---|
| | US - A - 3 991 485 (S.S. GOLENSKI)<br>* figures 1-10; colonne 2, ligne 6 - colonne 15, ligne 29 * | 1,4-10 |
| | US - A - 3 849 908 (J.L. STURGEON)<br>* figure 6; colonne 9, ligne 9 - colonne 10, ligne 18 * | 1 |
| | US - A - 3 859 738 (J. HEATON)<br>* figure 1; colonne 2, lignes 11-66 * | 1 |
| | US - A - 3 334 484 (R. TURGEON)<br>* en entier * | 1,2,5, 6,8,9 |
| A | US - A - 3 608 210 (P.H. O'SHEA) | 1 |
| A | FR - A - 1 437 324 (SICES) | 1 |
| A | FR - A - 2 183 448 (J. LEMOINE) | 1 |
| A | FR - A - 1 159 322 (A. SAPORTA) | 1 |

**CLASSEMENT DE LA DEMANDE (Int. Cl.³)**

G 09 B 19/16
A 61 B 5/18

**DOMAINES TECHNIQUES RECHERCHES (Int. Cl.³)**

G 09 B 19/16
G 09 B 9/08
A 61 B 5/18

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 18.05.1981 | CARDON |

OEB Form 1503.1   06.78